(19) Europäisches Patentamt
European Patent Office
Office européen des brevets

(11) Veröffentlichungsnummer: **0 347 781 B1**

# (12) EUROPÄISCHE PATENTSCHRIFT

(45) Veröffentlichungstag der Patentschrift: **16.02.94**

(51) Int. Cl.$^5$: **C07K 7/40**, A61K 37/26, C12N 15/00, //(C07K7/40,99:26)

(21) Anmeldenummer: **89111027.2**

(22) Anmeldetag: **17.06.89**

## (54) Mini-Proinsulin, seine Herstellung und Verwendung.

(30) Priorität: **23.06.88 DE 3821159**

(43) Veröffentlichungstag der Anmeldung:
**27.12.89 Patentblatt 89/52**

(45) Bekanntmachung des Hinweises auf die Patenterteilung:
**16.02.94 Patentblatt 94/07**

(84) Benannte Vertragsstaaten:
**AT BE CH DE ES FR GB GR IT LI LU NL SE**

(56) Entgegenhaltungen:
**EP-A- 0 132 769**
**EP-A- 0 163 529**
**EP-A- 0 195 691**

(73) Patentinhaber: **HOECHST AKTIENGESELLSCHAFT**

**D-65926 Frankfurt(DE)**

(72) Erfinder: **Dörschug, Michael, Dr.**
**Sonnenleite 20**
**D-4630 Bochum(DE)**
Erfinder: **Habermann, Paul, Dr.**
**Rossertstrasse 35**
**D-6239 Eppstein/Taunus(DE)**
Erfinder: **Seipke, Gerhard, Dr.**
**Wiesenstrasse 44**
**D-6238 Hofheim am Taunus(DE)**
Erfinder: **Uhlmann, Eugen, Dr.**
**Zum Talblick 31**
**D-6246 Glashütten/Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 347 781 B1

Rank Xerox (UK) Business Services
(3.10/3.09/3.3.3)

## Beschreibung

Die Erfindung betrifft ein neues "Mini-Proinsulin", bei dem die unverkürzte B-Kette nur über einen Argininrest an die A-Kette gebunden ist. Aus diesem Mini-Proinsulin ist Humaninsulin ohne aufwendige chemische Umsetzung zugänglich.

Mini-Proinsuline mit einer verkürzten C-Kette sind bekannt. So haben R. Wetzel et al., Gene 16 (1981), 63 - 71, ein Proinsulin mit einer auf sechs Aminosäuren verkürzten C-Kette beschrieben. Aus der europäischen Patentanmeldung mit der Publikationsnummer (EP-A) 0 055 945 sind entsprechende Proinsuline bekannt, deren C-Kette bis auf zwei Aminosäuren verkürzt ist.

Aus der EP-A 0 163 529 sind Insulinvorläufer mit einer verkürzten B-Kette bekannt, bei denen die C-Kette entweder ganz entfällt oder aber - bis auf eine Aminosäure - verkürzt ist. Diese Vorprodukte werden durch trypsinkatalysierte Transpeptidierung mit einem $\alpha$-Threoninester in reifes Humaninsulin überführt.

Demgegenüber betrifft die Erfindung das Human-Des-(32-65)-Proinsulin oder Mini-Proinsulin der Formel I

B(1-30)-Arg-A(1-21)     (I),

in der B(1-30) und A(1-21) die B- bzw. A-Kette des Humaninsulins bedeuten. Diese Verbindung dient nicht nur als Zwischenprodukt zur Herstellung von Human-Insulin-$Arg^{B31}$-OH, im folgenden "Mono-Arg-Insulin" genannt, das aus den europäischen Patentschriften (EP-B) 0 132 769 und 0 132 770 bekannt ist, sowie von Humaninsulin, sondern sie zeigt auch selbst eine gewisse Insulinaktivität.

Die Erfindung betrifft deshalb auch die Verbindung der Formel I zur Verwendung als Heilmittel, insbesondere zur Behandlung des Diabetes mellitus, und weiterhin Arzneimittel, enthaltend die Verbindung der Formel I, sowie Arzneimittel aus einem pharmakologisch unbedenklichen Träger und der Verbindung der Formel I.

Weiterhin betrifft die Erfindung die Verwendung der Verbindung der Formel I zur Herstellung des Mono-Arg-Insulins der Formel II

```
    S - S
    |   |
  A(1 - 21)
    |   |
    S   S                (II),
    |   |
    S   S
    |   |
  B(1 - 30) - Arg
```

in der A(1-21) und B(1-30) die vorstehend genannten Bedeutungen haben und die -S-S-Brücken wie im Insulin angeordnet sind, und von Humaninsulin, durch enzymatische Spaltung. Besonders vorteilhaft ist die unmittelbare Überführung der Verbindung der Formel I in Insulin in einer "Eintopfreaktion".

Die Erfindung betrifft weiterhin ein Verfahren zur Herstellung der Verbindung der Formel I, das dadurch gekennzeichnet ist, daß man eine für diese Verbindung codierende Genstruktur in einer Wirtszelle, vorzugsweise in einem Bakterium wie E. coli oder in einer Hefe, insbesondere Saccharomyces cerevisiae, exprimiert und, wenn die Genstruktur für ein Fusionsprotein codiert, die Verbindung der Formel I aus diesem Fusionsprotein freisetzt. Die Erfindung betrifft außerdem DNA-Sequenzen, die für die Verbindung der Formel I codieren, Genstrukturen oder Plasmide, die diese DHA enthalten, Sowie Wirtszellen, insbesondere Bakterien wie E. coli- oder Hefezellen, vor allem Hefen der Art Saccharomyces cerevisiae, die solche Genstrukturen oder Plasmide enthalten. Die Erfindung bezieht sich weiterhin auf Fusionsproteine, die eine Verbindung der Formel I enthalten, vorzugsweise Fusionsproteine, in denen die Verbindung der Formel I über das Brückenglied

- Met - Ile - Glu - Gly - Arg -

an den "Ballastanteil" des Fusionsproteins gebunden ist.

Weitere bevorzugte Ausgestaltungen der Erfindung werden im folgenden näher erläutert.

Die Figuren dienen zur Erläuterung der Beispiele, wobei Fig. 1 (und deren Fortsetzung in Fig. 1a und 1b) die Konstruktion der E. coli-Expressionsvektoren pIK10 und pSW3 und Fig. 2 (und deren Fortsetzung in Fig. 2a und 2b) die des Hefe-Expressionsvektors p$\alpha$fB102 bzw. p$\alpha$fB104 zeigt. Diese Vektoren codieren für

Mini-Proinsulin.

Es wurde gefunden, daß das Mini-Proinsulin die korrekte Faltung aufweist, so daß nach Spaltung mit Trypsin fast quantitativ Mono-Arg-Insulin entsteht. Es ergibt sich also ein überraschend einfaches Verfahren zur Herstellung von Mono-Arg-Insulin. Aus diesem kann in an sich bekannter Weise Humaninsulin hergestellt werden. Weiterhin dient Mono-Arg-Insulin als Wirkstoff in Arzneimitteln (EP-B 0 132 769).

In der EP-A 0 229 998 wurde der Expressionsvektor pK50 beschrieben. Mini-Proinsulin kann in Form eines Fusionsproteins entsprechend dieser Konstruktion in einem Bakterium wie E. coli hergestellt werden.

Das schwerlösliche Fusionsprotein kann durch Waschen mit neutralen Pufferlösungen angereichert werden. Durch Halogencyanspaltung (E. Gross und B. Wittkop, J. Am. Chem. Soc. 82 (1961) 1510 - 1517) wird das Mini-Proinsulin freigesetzt. Dieses liegt noch nicht in der biologisch aktiven Form vor, sondern besteht aus einer uneinheitlichen Mischung mit verschiedenen inter- und intramolekularen Disulfidbrücken, eventuell auch mit anderen Proteinfragmenten. Als chemisch einheitliches, relativ stabiles Derivat stellt man die S-Sulfonat-Form des Moleküls her (P. G. Katsoyannis et al., Biochemistry 6 (1967) 2635 - 2641). Dieses Derivat läßt sich sehr gut durch Ionenaustausch-Chromatographie reinigen und ist ein bewährtes Ausgangsmaterial für die Faltung in die native Raumstruktur unter Ausbildung der korrekten Disulfidbrücken (Y. C. Du et al., Sci. Sin. 15 (1965) 229 - 236; H. P. Gattner et al., Hoppe-Seylers Z. physiol. Chem. 362 (1981) 1943-1049; B. H. Frank et al. in: "Peptides: Synthesis-Structure-Function", D. H. Rich und E. Gross, Hrsg., (1981) 1043 - 1049). Der Erfolg dieser Faltung wird durch HPLC-Analyse der nach Spaltung mit S. aureus-Protease V8 entstehenden Fragmente abgesichert (U. Grau, Diabetes 34 (1985) 1174 - 1180).

Die Freisetzung von Mono-Arg-Insulin bzw. Insulin durch Einwirkung von Trypsin bzw. Carboxypeptidase B bzw. durch gleichwirkende Enzyme (W. Kemmler et al., J. Biol. Chem. 246 (1971) 2780 - 2795) verläuft ausgesprochen unkompliziert, denn es erweist ich sich hier als besonders vorteilhaft, daß die Zahl möglicher Spaltstellen gegenüber dem normalen Proinsulin reduziert ist. Dadurch ist die Spaltung erheblich einfacher (im Hinblick auf die Bildung von Nebenprodukten wie bei der Herstellung von Des-B30-Insulin oder Desocta-B23-B30-Insulin) zu steuern. Sowohl Mono-Arg-Insulin als auch Insulin lassen sich in bekannter Weise durch Ionenaustausch-Chromatographie in hochreiner Form isolieren. Die Bildung des Insulins und des Mono-Arg-Derivates, der Verlauf der Reinigung und die Qualität des Endproduktes werden mit üblichen RP-HPLC-Verfahren (G. Seipke et al., Angew. Chem. 98 (1986) 530 - 548) überprüft.

Überraschenderweise kommt es bei der Spaltung von Mini-Proinsulin im Gegensatz zu natürlichem Proinsulin kaum zur Bildung von Insulin-Des-B30. Da sich letzteres nur sehr schwer von Insulin trennen läßt, wird bei der Insulingewinnung aus natürlichem Proinsulin die "Zweitopfreaktion" bevorzugt, d. h. die Hauptprodukte der tryptischen Spaltung, Insulin-$Arg^{B31}$-$Arg^{B32}$ und Mono-Arg-Insulin, werden zunächst über Ionenaustauscher in bekannter Weise von Insulin-Des-B30 getrennt und anschließend mittels Carboxypeptidase B zu Humaninsulin gespalten (EP-B 0 195 691). Dagegen läßt sich das Mini-Proinsulin in idealer Weise in einer "Eintopfreaktion" unter gleichzeitiger Verwendung von Trypsin und Carboxypeptidase B bzw. mittels gleichwirkender Enzyme zu Humaninsulin umwandeln.

Die Expression der Verbindung der Formel I in Hefe mit anschließender Sekretion ist besonders vorteilhaft, da das korrekt gefaltete Proinsulinderivat direkt zu isolieren ist. Als Wirtssysteme wählt man Hefen, wie sie beispielsweise in der EP-A 0 248 227 aufgeführt sind, so z. B. Pichia pastoris, Hansenula polymorphis, Schizosaccharomyces pombe oder bevorzugt Saccharomyces cerevisiae.

Vektoren für die Expression in Hefen sind in großer Zahl bekannt. Die Herstellung des erfindungsgemäßen Insulinderivats wird im folgenden anhand des Hefe-$\alpha$-Faktorsystems beschrieben, was jedoch nur als beispielhaft zu verstehen ist, da in an sich bekannter Weise auch andere Expressionssysteme eingesetzt werden können.

Die Struktur des Hefe-Pheromongens MF$\alpha$ ist bekannt aus der Publikation Kurjan und Herskovitz, Cell 30 (1982) 933 - 943, wo auch die Möglichkeit der Expression anderer Gene und die Sekretion der Genprodukte diskutiert wird. Diesbezüglich kann auch auf Brake et al., Proc. Natl. Acad. Sci. USA 81 (1984), 4642 - 4646, verwiesen werden.

Alternativ kann ein Hefe-"Killertoxin"-System verwendet werden, oder die Sekretion über das System der sauren Phosphatase oder der Invertase ausgenutzt werden.

Als Hefevektoren werden vorteilhaft sogenannte "shuttle"-Vektoren verwendet, die einen bakteriellen Plasmid- und einen Hefeplasmid-Replikationsursprung sowie ein Gen oder Gene zur Selektion in beiden Wirtssystemen aufweisen. Ferner enthalten solche Vektoren die zur Expression fremder Gene notwendigen Promotorsequenzen und gegebenenfalls zur Verbesserung der Ausbeute eine Terminatorsequenz, so daß das heterologe Gen - zweckmäßig fusioniert an sekretorische Signale - zwischen Promotor und Terminator angeordnet ist. Solche Vektoren sind beispielsweise in der US-A 4 766 073 beschrieben.

Der genetische Code ist bekanntlich "entartet", d. h. daß nur für zwei Aminosäuren eine einzige Nukleotidsequenz codiert, während den restlichen 18 codierbaren Aminosäuren zwei bis sechs Tripletts

zuzuordnen sind. Für die Synthese des Gens für das Mini-Proinsulin steht somit eine große Vielfalt von Codon-Kombinationen zur Auswahl. Es wurde nun gefunden, daß die für Mini-Proinsulin codierende DNA-Sequenz I (die im Anhang in Form der beiden Genfragmente IK I (Tabelle 1) und IK II (Tabelle 2) wiedergegeben ist) besonders vorteilhaft ist, da sie auf den Codongebrauch sowohl von Hefe als auch von E. coli optimiert ist.

Am 5′-Ende des codierenden Stranges der DNA-Sequenz I befindet sich eine "uberhängende" DNA-Sequenz, entsprechend der Restriktionsendonuklease KpnI. Am 3′-Ende des codierenden Stranges ist dagegen die einzelsträngige Sequenz entsprechend dem Restriktionsenzym HindIII überhängend. Diese beiden unterschiedlichen Erkennungssequenzen gewährleisten die Insertion der DNA-Sequenz I in Plasmide in der gewünschten Orientierung. Der codierenden Sequenz folgen auf das Triplett Nr. 65 für Asparagin zwei Translations-Terminationscodons (Stop-Codons). Eine interne singuläre Schnittstelle für das Restriktionsenzym PstI (Codon 41/42) ermöglicht die Subklonierung zweier Genfragmente, die in gut untersuchte Plasmide wie pUC18 oder Derivate dieser Plasmide eingebaut werden können.

Zusätzlich wurden innerhalb des Strukturgens eine Reihe von weiteren singulären Erkennungssequenzen für Restriktionsenzyme eingebaut, die einerseits einen Zugang zu Teilsequenzen des Proinsulins schaffen und andererseits die Durchführung von Mutationen erlauben:

| Restriktionsenzym | Schnitt nach Nukleotid Nr. (codierender Strang) |
|---|---|
| AccI | 201 |
| DraIII | 46 |
| FnuDII | 107 |
| HgaI | 105 |
| HindIII | 213 |
| HinfI | 17 |
| HpaI | 22 |
| HphI | 76 |
| MaeI | 155 |
| MaeIII | 191 |
| MboII | 89 |
| MluI | 106 |
| NcoI | 207 |
| NlaIII | 208 |
| PvuII | 175 |
| SalI | 201 |
| SpeI | 154 |
| StyI | 207 |
| TaqI | 202 |

Die DNA-Sequenz I wurde in wesentlichen Punkten von der natürlichen Sequenz abgeändert. Dadurch war die Einführung der zahlreichen singulären Schnittstellen für Restriktionsenzyme möglich.

Die DNA-Sequenz I läßt sich aus insgesamt 6 Oligonukleotiden mit einer Kettenlänge von 47 bis 96 Nukleotideinheiten aufbauen. Dabei verfährt man wie nachfolgend beschrieben.

Das Genfragment IK I (Tabelle 1) läßt sich aus 4 Oligonukleotiden mit einer Kettenlänge von 47 bis 74 Einheiten aufbauen, indem diese zunächst chemisch synthetisiert und dann über "sticky ends" von 3 Nukleotiden enzymatisch verknüft werden. Die sticky ends entsprechen denen des Restriktionsenzyms DraIII, was für spätere Modifikationen von Vorteil ist.

Das Genfragment IK II (Tabelle 2) ist aus zwei chemisch synthetisierten Oligonukleotiden mit einer Länge von 88 und 96 Nukleotideinheiten zu erhalten.

Beispiel 1

a) Chemische Synthese eines einzelsträngigen Oligonukleotids

Am Beispiel des Oligonukleotids Nr. 4 (Tabelle 1) wird die Synthese der DNA-Bausteine erläutert. Für die Festphasensynthese wird das am 3′-Ende stehende Nukleosid, im vorliegenden Falle also Adenin (Nukleotid Nr. 125), über die 3′-Hydroxyfunktion kovalent an einen Träger gebunden verwendet. Trägermaterial ist mit langkettigen Aminoalkylresten funktionalisiertes CPG ("Controlled Pore Glass").

In den folgenden Syntheseschritten wird die Basenkomponente als 5′-O-Dimethoxytritylnucleosid-3′-phosphorigsäure-β-cyanoethylester-dialkylamid eingesetzt, wobei das Adenin als $N^6$-Benzoyl-Verbindung, das Cytosin als $N^4$-Benzoyl-Verbindung, das Guanin als $N^2$-Isobutyryl-Verbindung und das Thymin ohne Schutzgruppe vorliegen.

25 mg des polymeren Trägers, der 0,2 µmol 5′-O-Dimethoxytrityl-$N^4$-Benzoyl-2′-desoxyadenosin gebunden enthält, werden nacheinander mit folgenden Agentien behandelt:

A) Acetonitril
B) 3 % Trichloressigsäure in Dichlormethan
C) Acetonitril
D) 5 µmol des entsprechenden Nukleosid-3′-O-phosphits und 25 µmol Tetrazol in 0,15 ml wasserfreiem Acetonitril
E) Acetonitril
F) 20 % Acetanhydrid in Tetrahydrofuran mit 40 % Lutidin und 10 % Dimethylaminopyridin
G) Acetonitril
H) 3 % Jod in Lutidin/Wasser/Tetrahydrofuran im Volumenverhältnis 5:4:1

Unter "Phosphit" wird hierbei der 2′-Desoxyribose-3′-mono-phosphorigsäure-mono-β-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden.

Nach abgeschlossener Synthese erfolgt die Abspaltung der Dimethoxytritylgruppe wie in A) bis C) beschrieben. Durch die Behandlung mit Ammoniak wird das Oligonukleotid vom Träger gespalten und zugleich werden die β-Cyanoethylgruppen eliminiert. Eine 16-stündige Behandlung der Oligomeren mit konzentriertem Ammoniak bei 50 °C spaltet die Aminoschutzgruppen der Basen quantitativ ab. Das so erhaltene Rohprodukt wird durch Polyacrylamid-Gelelektrophorese gereinigt.

In analoger Weise werden auch die Oligonukleotide 1 - 3 (Tabelle 1), 5 und 6 (Tabelle 2) hergestellt.

b) Enzymatische Verknüpfung der einzelsträngigen Oligonukleotide

Zur enzymatischen Phosphorylierung der Oligonukleotide am 5′-Terminus werden je 1 µmol der Oligonukleotide 1 und 4 mit 5 µmol Adenosintriphosphat mit vier Einheiten T4 Polynukleotid-Kinase in 20 µl 50 mM Tris-HCl-Puffer (pH 7,6), 10 mM Magnesiumchlorid und 10 mM Dithiothreitol (DTT) 30 Minuten bei 37 °C behandelt. Das Enzym wird durch fünfminütiges Erhitzen auf 95 °C desaktiviert. Die Oligonukleotide 2 und 3, welche die "überhängenden" einzelsträngigen Sequenzen bilden, werden nicht phosphoryliert. Dies verhindert bei der nachfolgenden Ligation die Ausbildung größerer Genfragmente.

Die Oligonukleotide 1 bis 4 werden wie folgt ligiert: Je 1 µmol der Oligonukleotide 1 und 2 bzw. 3 und 4 werden paarweise hybridisiert, indem diese in jeweils 20 µl 50 mM Tris-HCl-Puffer (pH 7,6), 10 mM Magnesiumchlorid und 10 mM DTT gelöst werden, diese Lösung 5 Minuten auf 95 °C erhitzt und binnen 2 Stunden auf Raumtemperatur abgekühlt wird. Dabei werden die Oligonukleotide 1 und 4 in Form ihrer 5′-Phosphate eingesetzt. Zur weiteren Verknüpfung der gebildeten bihelikalen DNA-Fragmente werden die Lösungen derselben vereinigt, 15 Minuten auf 60 °C erwärmt und auf Raumtemperatur abgekühlt. Dann setzt man 2 µl 0,1 M DTT, 16 µl 2,5 mM Adenosintriphosphat (pH 7) sowie 1 µl T4 DNA-Ligase (400 units) zu und inkubiert 16 Stunden bei 22 °C.

Die Reinigung der so erhaltenen Genfragmente (Tabellen 1 und 2) erfolgt durch Gelelektrophorese auf einem 10 %igen Polyacrylamidgel (ohne Harnstoffzusatz, 40 x 20 x 0,1 cm), wobei als Markersubstanz ØX174 DNA (Fa. BRL), geschnitten mit HinfI, oder pBR322, geschnitten mit HaeIII, dient.

**Beispiel 2**

a)Klonierung der synthetisierten DNA-Fragmente

Das handelsübliche Plasmid pUC19 wird mit den Restriktionsenzymen KpnI und PstI geöffnet und das große Fragment (1) über ein 0,8 %iges "Seaplaque"-Gel abgetrennt. Dieses Fragment wird mit der synthetischen DNA (2) gemäß Tabelle 1 mit T4 DNA-Ligase umgesetzt und das Ligationsgemisch mit kompetenten E. coli 79/02-Zellen inkubiert. Das Transformationsgemisch wird auf IPTG/Xgal-Platten, die 20 mg/l Ampicillin enthalten, ausplattiert. Aus den weißen Kolonien wird die Plasmid-DNA isoliert und durch Restriktions- und DNA-Sequenzanalyse charakterisiert. Die gewünschten Plasmide erhalten die Bezeichnung pIK1.

Entsprechend wird die DNA (5) nach Tabelle 2 in pUC19 ligiert, das mit PstI und HindIII geöffnet wurde (4). Man erhält das Plasmid pIK2 (6).

b) Konstruktion des Mini-Proinsulin-Gens

Aus den Plasmiden pIK1 (3) und pIK2 (6) werden die DNA-Sequenzen (2) und (5) gemäß Tabelle 1 und 2 reisoliert und mit pUC19 ligiert, das mit KpnI und HindIII geöffnet wurde (7). Man erhält so das Plasmid pIK3 (8), das für eine modifizierte Humaninsulinsequenz codiert.

Das Plasmid pIK3 (8) wird mit MluI und SpeI geöffnet und das große Fragment (9) isoliert. Dieses wird mit der DNA-Sequenz (10)

```
      B30         A1   A2   A3   A4   A5   A6   A7   A8   A9
    (Thr)(Arg) Gly  Ile  Val  Glu  Gln  Cys  Cys (Thr)(Ser)      (10)
 5´   CG  CGT  GGT  ATC  GTT  GAA  CAA  TGT  TGT  A          3´
 3´         A  CCA  TAG  CAA  CTT  GTT  ACA  ACA  TGA  TC   5´
    (MluI)                                                    (SpeI)
```

ligiert, die das letzte Codon der B-Kette (B30) um ein Arginin-Codon ergänzt und die herausgeschnittenen Codons für die ersten 7 Aminosäuren der A-Kette ersetzt und die Codons Nür die Aminosäuren 8 und 9 dieser Kette ergänzt.

Man erhält so das Plasmid pIK4 (11), das für das erfindungsgemäße Human-Mini-Proinsulin codiert.

c) Expressionsvektoren für Mini-Proinsulin

**pIK I:**

Das aus der EP-A 0 229 998 bekannte Plasmid pK50 (12) (dort Beispiel 3; Figur 3 (33)) wird mit EcoRI und HindIII gespalten. Beide Fragmente (13) und (14) werden isoliert. Das kleine Fragment (14) mit der IL-2-Teilsequenz wird mit MluI nachgespalten und die IL-2-Teilsequenz (15) isoliert.

Das Plasmid pIK4 (11) wird mit EcoRI und HpaI gespalten und das große Fragment (16) isoliert. Dieses wird nun mit der IL-2-Teilsequenz (15) und der synthetischen DNA (17)

```
                                            B1   B2
                    Met  Ile  Glu  Gly  Arg  Phe  Val
 5´   CG   CGT  ATG  ATT  GAG  GGC  CGT  TTC  GTT   3´   (17)
 3´          A  TAC  TAA  CTC  CCG  GCA  AAG  CAA   5´
    (MluI)                                    (HpaI)
```

ligiert, wobei man das Plasmid pIK8 (18) erhält. Dieses codiert für ein Fusionsprotein, in dem auf die ersten 38 Aminosäuren des IL-2 ein Brückenglied Met-Ile-Glu-Gly-Arg und hierauf die Aminosäuresequenz des Mini-Proinsulin folgen.

Aus dem Plasmid pIK8 (18) wird das EcoRI-HindIII-Fragment (19) herausgeschnitten, das für das genannte Fusionsprotein codiert. Dieses Fragment wird mit dem großen Fragment (13) ligiert, das bei der Spaltung von pK50 erhalten wurde. Man erhält so den Expressionsvektor pIK10 (20), der für das vorstehend charakterisierte Fusionsprotein codiert.

pSW3:

Entfernt man aus dem Vektor pIK10 (20) das NdeI-BstEII-Segment, das die "bom site" einschließt, so erhält man einen Vektor, der in höherer Kopienzahl in der Zelle vorliegt und - wegen der fehlenden "bom

site" durch konjugative Plasmide nicht mehr mobilisierbar ist.

Hierzu wird der Vektor pIK10 (20) mit BstEII und NdeI geschnitten, die DNA mit Ethanol gefällt, in DNA-Polymerase-Puffer überführt und einer Klenow-Polymerasereaktion unterworfen. Die so entstandenen stumpfendigen DNA-Fragmente werden gelelektrophoretisch aufgetrennt und das größere Fragment (21) isoliert. Durch Ligierung erhält man den Vektor pSW3 (22). Nach Transformation von kompetenten E. coli-Mc1061-Zellen und Amplifikation wird das Plasmid pSW3 (22) isoliert und charakterisiert.

Beispiel 3: Expression in dem Stamm E. coli W3110

Eine Übernachtkultur aus E. coli-Zellen, die das Plasmid pIK10 (20) oder pSW3 (22) enthalten, wird mit LB-Medium (J. H. Miller, Experiments in Molecular Genetics, Gold Spring Harbor Laboratory, 1972), das 50 µg/ml Ampicillin enthält, im Verhältnis von etwa 1:100 verdünnt und das Wachstum über OD-Messung verfolgt. Bei OD = 0,5 wird die Kultur auf 1 mM IPTG eingestellt und die Bakterien nach 150 bis 180 Minuten abzentrifugiert. Die Bakterien werden 5 Minuten in einer Puffermischung (7 M Harnstoff, 0,1 % SDS, 0,1 M Natriumphosphat, pH 7,0) gekocht und Proben auf eine SDS-Gelelektrophoreseplatte aufgetragen. Nach gelelektrophoretischer Analyse wird im Bereich von ca. 10 Kd eine zusätzliche Bande beobachtet, die dem erwarteten Fusionsprotein entspricht. Diese Bande reagiert im "Western Blot"-Experiment mit gegen Insulin gerichteten Antikörpern.

Schließt man die Zellen unter Druck auf und zentrifugiert anschließend den Aufschluß ab, so wird das Fusionsprotein im Sediment neben anderen unlöslichen Zellbestandteilen gefunden.

Die angegebenen Induktionsbedingungen gelten für Schüttelkulturen; bei größeren Fermentationen ist die Wahl anderer Medien und Bedingungen, z. B. zur Erzielung veränderter O.D.-Werte, zweckmäßig.

Beispiel 4:

a) Herstellung von Mono-Arg-Insulin

40 g des durch Zentrifugation und Waschen mit Phosphatpuffer (pH 7) bzw. Wasser angereicherten Fusionsproteins (Trockensubstanzgehalt ca. 25 %) werden in 75 ml 98 - 100 %iger Phosphorsäure gelöst und mit 5 g BrCN versetzt. Nach 6-stündiger Reaktion bei Raumtemperatur wird das Gemisch mit 2 l Wasser versetzt und gefriergetrocknet.

Das Fragmentgemisch (10 g) wird in 1 l Pufferlösung (8 M Harnstoff, 0,2 M Tris-HCl (pH 8,5)) gelöst, auf 30 °C erwärmt und mit 10 g Natriumsulfit und 2,5 g Natriumtetrathionat versetzt. Nach 90 Minuten bei 30 °C gibt man 3 l kaltes Wasser zu und stellt den pH-Wert auf 7,0 ein. Die entstehende Flockung wird abzentrifugiert. Das Hexa-S-Sulfonat des Mini-Proinsulins wird aus dem Überstand durch pH-Einstellung auf 3,5 ausgefällt. Nach 15 Stunden Inkubation bei +4 °C wird zentrifugiert. Der Niederschlag wird mit 200 ml Wasser gewaschen und gefriergetrocknet. Man erhält 4,8 g eines Substanzgemisches, in dem durch RP-HPLC ein Mini-Proinsulinanteil von 900 mg bestimmt wird. Die Anreicherung des S-Sulfonats erfolgt in 2 Stufen:

1. Anionenaustausch-Chromatographie über eine 5 x 60 cm-Säule mit ®Fractogel TSK DEAE-650 M in 3 M Harnstoff; 0,05 M Tris-HCl (pH 8,3). Die Elution wird mit einem Gradienten von 0,05 - 0,5 M NaCl (je 6 l) vorgenommen. Nach Analyse des Eluats durch isoelektrische Fokussierung fällt man das Produkt aus den vereinigten Fraktionen durch Verdünnung auf 1 M Harnstoff und pH-Einstellung auf 3,5 aus.
2. Entfernung von hoch- und niedermolekularen Verunreinigungen durch Gelfiltration über ®Sephacryl S200 in 3 M Harnstoff; 0,05 M Tris-HCl; 0,05 M NaCl (pH 8,3). Analyse der Fraktionen und Isolierung des Produktes werden wie im vorhergehenden Schritt durchgeführt. Das Präzipitat wird mit 20 ml Wasser gewaschen und gefriergetrocknet. Man erhält 1,10 g des auf 69 % Reinheit angereicherten Produkts.

Zur Faltung und Disulfidbrückenbildung wird das S-Sulfonat in 50 ml 8 M Harnstoff; 0,02 M Tris-HCl bei pH 8,6 gelöst. Nach Zusatz einiger Tropfen Octanol wird 15 Minuten lang nachgereinigter Stickstoff eingeleitet. Durch Zugabe von 1,1 ml (16 mMol) 2-Mercaptoethanol erfolgt innerhalb 1 Stunde bei Raumtemperatur die vollständige Reduktion. Die Lösung wird auf eine ®Sephadex G25-Säule (5 x 60 cm) aufgetragen und mit 0,05 M Glycin/NaOH (pH 10,6) eluiert. Die Proteinfraktion in 300 ml des Glycinpuffers wird nach Kontrolle und eventueller Korrektur des pH-Wertes (10,6) 2 Tage bei 4 °C aufbewahrt. Danach wird der pH-Wert auf 6,8 eingestellt und die Lösung mit 1 mg (3,5 U) Trypsin (Merck, mit L-1-p-Tosylamino-2-phenylethyl-chlormethylketon (TPCK) behandelt) bei Raumtemperatur für 4 Stunden inkubiert. Anschließend wird der pH-Wert auf 3,5 eingestellt, die Lösung mit 1 mg Trypsin-Inhibitor aus Sojabohne (Sigma) und 3 ml 10 % $ZnCl_2$ versetzt und wieder auf pH 6,8 zurückgestellt. Der entstandene Niederschlag wird durch Zentrifugation abgetrennt. Er enthält überwiegend Mono-Arg-Insulin, das durch Ionenaustausch-Chromatographie an S-Sepharose® (2,5 x 40 cm) in einem Puffer aus 50 mM Milchsäure, 30 % Isopropanol (pH 3,5) gereinigt wird. Die Elution erfolgt mittels eines Gradienten von 0,05 - 0,50 M NaCl (je 1 l). Das Eluat wird durch HPLC analysiert; aus den produkthaltigen Fraktionen fällt man das Mono-Arg-Insulin nach 1:1-Verdünnung mit $H_2O$ durch Zusatz von 10 ml 10 % $ZnCl_2$ pro 1 l und pH-Einstellung auf 6,8 aus. Die durch Zentrifugation abgetrennte Fällung wird aus einem Puffer aus 1 g/l Phenol, 10,5 g/l Citronensäure und 200 mg/l $ZnCl_2$ bei pH 6 kristallisiert. Man erhält nach Gefriertrocknung der mit etwas Wasser gewaschenen Kristalle 390 mg Mono-Arg-Insulin in über 90 % Reinheit.

Beispiel 5:

Herstellung von Insulin

200 mg Mono-Arg-Insulin (s. Beispiel 4) werden in 100 ml 0,05 M Tris-HCl (pH 8,5) gelöst. Dann wird 1 U (ca. 4 µg) Carboxypeptidase B zugesetzt und die Lösung bei Raumtemperatur schwach gerührt. Nach 3 Stunden wird das Humaninsulin durch Ansäuern auf pH 3,5 und Zugabe von 1 ml 10 % $ZnCl_2$ bei pH 5,5 kristallisiert. Man erhält 200 mg kristallines Insulin mit einer Reinheit von mehr als 85 %. Dieses Material wird einer Reinigung durch Ionenaustauscher-Chromatographie an einer Säule mit Fractogel TSK-DEAE-650 M (2,5 x 40 cm) in 0,1 % ®Lutensol ON 100 (BASF AG; Oxethylat eines linearen, gesättigten Fettalkohols von im wesentlichen 12 C-Atomen); 0,05 M Tris-HCl (pH 8,3) unterzogen, wobei die Elution mit einem Gradienten von 0 - 0,4 M NaCl (je 1 l) erfolgt. Aus den mittels HPLC identifizierten produkthaltigen Fraktionen wird das Insulin nach Zusatz von 10 ml 10 % $ZnCl_2$ und 1 ml 10 % Citronensäure bei pH 5,5 kristallisiert. Nach langsamem Rühren über Nacht wird zentrifugiert und das erhaltene Sediment aus 20 ml eines Puffers aus 5 g/l Citronensäure, 125 ml/l Aceton und 200 mg/l $ZnCl_2$ bei pH 5,5 umkristallisiert. Man erhält 160 mg Insulin mit einer Reinheit von mehr als 95 %.

Beispiel 6:

Herstellung von Insulin aus Mono-Arg-Insulin durch kombinierten Einsatz von Trypsin und Carboxypeptidase B

5 mg Mono-Arg-Insulin werden in 20 ml 0,1 M Tris-HCl (pH 8,0) gelöst und auf 30 °C erwärmt. Es werden gleichzeitig 2,5 µl Trypsinlösung (1 U/ml) und 150 µl Carboxypeptidase B-Lösung (1 U/ml) zugegeben. Nach 3 Stunden wird die Lösung auf pH 3,5 eingestellt und 2,5 µl Trypsininhibitorlösung (1 U/ml) sowie 200 µl 10 %ige $ZnCl_2$-Lösung zugegeben. Das Humaninsulin wird durch Einstellen auf pH 6,8 ausgefällt, abzentrifugiert und wie in Beispiel 5 kristallisiert. Das kristallisierte Insulin hat eine Reinheit > 95 %.

Beispiel 7: Konstruktion eines Hefe-Expressionsvektors

Zunächst wird die DNA-Sequenz (23) (Tabelle 3) nach dem Phosphitverfahren synthetisiert. Diese DNA-Sequenz (23) codiert für die Aminosäuren 49 bis 80 des MFα-Vorläuferproteins und entspricht im wesentlichen der natürlichen DNA-Sequenz.

Die DNA-Sequenz (23) wird zunächst als Sonde zur Isolierung des Gens für den α-Faktor verwendet und hierzu mit $^{32}P$ markiert. Mit Hilfe dieoer Sonde wird aus einer genomischen λgt11-Hefegenbank (wie sie inzwischen handelsüblich und z. B. bei Clontech Laboratories Inc., 4055 Fabian Way, Palo Alto, CA94303 erhältlich sind) isoliert. Dazu werden λgt11-Phagen, die das α-Faktorgen tragen, in einem Plaque-Hybridisierungsexperiment identifiziert. Phagen aus als positiv identifizierten Plaques werden isoliert, vermehrt und die DNA gewonnen. Diese wird mit EcoRI gespalten und auf einem 0,8 %-igen Agarosegel analysiert. Nach einem "Southern transfer"-Experiment wird die Membran gegen die $^{32}P$-markierte DNA-Sequenz (23) hybridisiert. Phagen-DNA, die ein ca. 1,75 kb-Fragment (24) aufweist, das gegen die DNA-Sequenz (23) hybridisiert, wird erneut mit dem Enzym gespalten und das entsprechende Fragment (24) isoliert. Der Vektor pUC 19 wird mit EcoRI geöffnet (25) und mit dem 1,75 kb-Fragment (24) mit T4-Ligase umgesetzt. Man erhält den Klonierungsvektor (26).

Mit dem Ligationsgemisch wird der Stamm E. coli 79/02 transformiert. Weiße Kolonien werden isoliert, hieraus die Plasmid-DNA gewonnen und Plasmide (26), die das 1,75 kb-EcoRI-Fragment enthalten, identifiziert.

Die natürliche DNA-Sequenz des Vorläuferproteins für MFα enthält im Bereich der Aminosäuren 8 bis 10 eine PstI-Schnittstelle und im Bereich der Aminosäuren 48/49 eine TaqI-Schnittstelle. Aus der isolierten

Plasmid-DNA (26) wird nun durch Umsetzung mit PstI und TaqI das Fragment (27) isoliert, das für die Aminosäuren 9 bis 48 der MFα-Vorläufersequenz codiert. Der Vektor pUC18 wird mit PstI und KpnI geöffnet (28) und mit dem PstI-TaqI-Fragment (27) sowie mit der synthetischen DNA-Sequenz (23) mit Hilfe von T4-Ligase umgesetzt. Mit dem Ligationsgemisch wird E. coli 79/02 transformiert. Das Transformationsgemisch wird auf IPTG-Xgal-Ap-Platten ausplattiert. Weiße Kolonien werden isoliert und die Plasmid-DNA dieser Klone durch Restriktionsanalyse charakterisiert. Man erhält so den Klonierungsvektor (29), der für die Aminosäuren 8 bis 80 der MFα-Vorläufersequenz codiert.

Aus dem Klonierungsvektor (29) wird durch Umsetzung mit PstI und KpnI die genannte codierende Sequenz (30) ausgeschnitten und in die im folgenden beschriebene Ligierung eingebracht. Hierzu wird der Klonierungsvektor (26) mit EcoRI und partial mit PstI umgesetzt und das die Codierungssequenz für die ersten 8 Aminosäuren der MFα-Vorläufersequenz umfassende Fragment (31) isoliert. Weiterhin wird der Vektor pUC19 mit EcoRI und KpnI geöffnet (32) und mit den beiden beschriebenen Fragmenten (30) und (31) ligiert, wobei der Klonierungsvektor (33) entsteht. Dieser codiert für die gesamte Vorläufersequenz des MFα bis zur Aminosäure 80.

Der Klonierungsvektor (33) wird mit KpnI und HindIII geöffnet und das große Fragment (34) isoliert. Dieses wird mit dem KpnI-HindIII-Fragment (35) aus dem Plasmid (11), das für das Mini-Proinsulin codiert, ligiert. Man erhält so das Plasmid pIK20 (36), dessen Struktur durch Restriktionsanalyse bestätigt wird.

Das Plasmid Yep13 (Broach et al., Gene 8 (1979) 121) wird mit BamHI geöffnet und die überstehenden Enden mit Klenow-Polymerase aufgefüllt (38). Die DNA wird mit Ethanol gefällt und mit alkalischer Rinderphosphatase behandelt.

Aus dem Klonierungsvektor (36) wird mit HindIII und EcoRI das für das Insulin-Derivat und die Vorläufersequenz des MFα codierende Fragment ausgeschnitten und die überstehenden Enden wie beschrieben aufgefüllt (37).

Die beiden stumpfendigen DNA-Sequenzen (37) und (38) werden miteinander ligiert, wobei die Plasmide pαfB102 (39) und pαfB104 (40) entstehen. Diese beiden Plasmide unterscheiden sich nur in der Orientierung des insertierten Fragmentes.

Nie in der EP-A 0 171 024 beschrieben, kann hinter die insertierte Sequenz ein Terminator eingesetzt werden (Figuren 4 bis 6 der EP-A 0 171 024). Hierfür eignen sich die NcoI- und/oder die BamHI-Schnittstellen.

Nach Amplifikation der Plasmid-DNA in E. coli MM294 wird das Plasmid pαfB102 (39) in die Leucin-bedürftigen Hefestämme Y79 (α,trp1-1,leu2-1) (Cantrell et al., Proc. Acad. Natl. Sci. USA 82 (1985) 6250) und DM6-6(α/α leu2-3,112::ura3$^+$/leu2::lys2$^+$, trp1$^-$/trp1$^-$, his3-11, 15/his3-11, 15, ura3$^-$/ura3$^-$, lys2$^-$/lys2$^-$, arg4-17/arg4$^+$, ade1$^-$/ade1$^+$) (Maya Hanna, Dept. Mol. Biol. Massachusetts General Hospital, Boston, USA) nach der Lithium-Methode von Ito, H. et al., J. Bacteriol., 153 (1983) 163 transformiert. Kolonien, die auf selektivem Medium ohne Leucin-Zusatz wachsen können, werden isoliert und vereinzelt. Hefe-Minimalmedium wird mit den einzelnen Kolonien beimpft und 24 Stunden bei 28°C inkubiert. Die Zellen werden abzentrifugiert und der Überstand in einem RIA-Test auf Insulinaktivität überprüft. Aus Hefeklonen, deren Überstand Insulinaktivität zeigt, wird die Plasmid-DNA reisoliert und durch Restriktionsanalyse charakterisiert. Die transformierten Hefestämme werden für die folgende Expression eingesetzt.

Beispiel 8: Expression in Hefe

10 ml Hefevollmedium wird mit Zellen, die aus einer frischen Übernachtkultur eines nach Beispiel 7 erhaltenen Stammes aus selektivem Medium entnommen wurden, so beimpft, daß eine optische Dichte $OD_{600}$ = 0,1 erreicht wird. Die Kultur wird 8 Stunden bei 28°C geschüttelt, worauf 90 ml frisches Medium zugesetzt werden. Anschließend wird die Kultur für weitere 20 Stunden geschüttelt. Die Zellen werden abzentrifugiert und die Insulinkonzentration im Überstand bestimmt. Die Bedingungen ändern sich für größere Fermentation, z. B. kann frisches Medium kontinuierlich zugesetzt werden.

Beispiel 9: Reinigung von Mono-Arg-Insulin aus Hefeüberstand

Der Fermentationsüberstand wird über eine Adsorptionssäule mit einem porösen Adsorberharz aus einem Copolymer von Styrol und Divinylbenzol (®Diaion HP20) gegeben. Die Säule wurde zuvor mit einem 20 - 50 mM Acetatpuffer (pH 5) äquilibriert. Nach Waschen mit Tris-Puffer (pH 8) wird ein Isopropanolgradient (0 - 50 %) mit dem 10-fachen Säulenvolumen angelegt. Insulinhaltige Fraktionen werden auf pH 6 eingestellt, mit ®MATREX CELLUFINE AM (Fa. Amicon) versetzt, gerührt und abgenutscht und mit 50 mM Acetatpuffer (pH 6) nachgewaschen. Die Waschfraktion und die Hauptfraktion werden vereinigt, mit Milchsäure auf pH 3,5 eingestellt und über eine S-SEPHAROSE-Säule, die mit 50 mM Milchsäure (pH 5)/30

% Isopropanol äquilibriert wurde, gegeben.

Die Elution erfolgt mittels eines 0 - 0,6 M NaCl-Gradienten. Mini-Proinsulin eluiert im Bereich 0,25 - 0,3 M.

Die proinsulinhaltigen Fraktionen werden auf 1/4 des Volumens eingeengt und über eine Säule mit Biogel-P10 (Bio-Rad), äquilibriert in 6 % Essigsäure (pH 2), gegeben. Das insulinhaltige Eluat wird lyophilisiert und über einen präparativen "reversed phase"-HPLC-Schritt (RP18-Material, 0,1 % TFA, Acetonitrilgradient 20 - 40 %) gereinigt. Nach anschließender Gefriertrocknung wird in Tris-Puffer (pH 6,8) gelöst und mit 4 Einheiten Trypsin pro Gramm Mono-Arg-Proinsulin bei Raumtemperatur 3 bis 5 Stunden inkubiert. Der Reaktionsverlauf wird über "reversed-phase"-Analytik kontrolliert. Es zeigt sich, daß nahezu quantitativ Mono-Arg-Insulin entsteht. Am Ende der Reaktion wird ein pH-Wert von 3,5 eingestellt und die Reaktion durch Zugabe einer äquivalenten Menge Trypsininhibitor beendet. Anschließend wird eine Zinkchloridkonzentration von 0,21 g/l und ein pH-Wert von 6,8 eingestellt. Man erhält einen flockigen Niederschlag, der in Milchsäurepuffer gelöst wird. Die Komponenten werden über S-SEPHAROSE-Chromatographie voneinander getrennt. Fraktionen, die Mono-Arg-Insulin enthalten, werden vereinigt und mit Wasser im Verhältnis 1:1 gemischt. Anschließend wird die Lösung mit 10 ml 10 % $ZnCl_2$ pro 1 l versetzt. Bei pH 6,8 fällt dann Mono-Arg-Insulin aus, das dann in (für Insulin) bekannter Weise umkristallisiert wird.

Beispiel 10: Herstellung von Humaninsulin

Das nach Beispiel 9 dargestellte Mono-Arg-Insulin dient als Ausgangssubstanz für die Carboxypeptidase B-Spaltung. Dazu wird das Insulinderivat in Tris-Puffer (pH 8,5) gelöst und mit 5 Einheiten Carboxypeptidase B pro Gramm Mono-Arg-Insulin versetzt. Unter schwachem Rühren bei Raumtemperatur wird die Reaktion über 3 Stunden durchgeführt. Dann wird wie in Beispiel 9 beschrieben mit $ZnCl_2$ gefällt. Humaninsulin wird dann in bekannter Weise gereinigt (DE-B 2 629 568).

Tabelle 1: Genfragment IK I (2)

```
                        B1
                        Phe
           10           20           30            40
            .            .            .             .
     <------------------------------2---------------------------
     CT TTG GAC AAG AGA TTC GTT AAC CAA CAC TTG TGT GGT TCT CAC
CAT GGA AAC CTG TTC TCT AAG CAA TTG GTT GTG AAC ACA CCA AGA GTG
<-----------------------------1------------------------------->
(KpnI)                      HpaI


50           60            70           80           90
 .            .             .            .            .
--> <----------------------------------------------4--------
TTG GTG GAA GCG TTG TAC TTG GTT TGT GGT GAG CGT GGT TTC TTC
AAC CAC CTT CGC AAC ATG AAC CAA ACA CCA CTC GCA CCA AAG AAG
<----------------------------------------------3------------

                 B30
                 Thr Arg Lys Gly Ser Leu
      100           110          120
       .             .            .
 ---------------------------------------->
TAC ACT CCA AAG ACG CGT AAG GGT TCT CTG CA
ATG TGA GGT TTC TGC GCA TTC CCA AGA G
 ---------------------------------->
                 MluI              (PstI)
```

Tabelle 2: Genfragment IK II (5)

```
                  A1
     Gln Lys Arg Gly

           130          140          150           160
            .            .            .             .
       <-----------------------------------------------------6
       G AAG CGT GGT ATC GTT GAA CAA TGT TGT ACT AGT ATC TGT TCT
  AC GTC TTC GCA CCA TAG CAA CTT GTT ACA ACA TGA TCA TAG ACA AGA
 <-----------------------------------------------------------5
(PstI)                                         SpeI


                                A21
                                Asn
170          180           190           200          210
 .            .             .             .            .
------------------------------------------------------->
TTG TAC CAG CTG GAA AAC TAC TGT AAC TGA TAG TCG ACC CAT GGA
AAC ATG GTC GAC CTT TTG ATG ACA TTG ACT ATC AGC TGG GTA CCT TCG A
------------------------------------------------------------->
                                                           (HindIII)
```

**Tabelle 3: DNA-Sequenz (23)**

```
                     50                        55
5'       C GAT GTT GCT GTT TTG CCA TTC TCC
3'         TA CAA CGA CAA AAC GGT AAG AGG
   (TaqI)
                         60                    65
    AAC AGT ACT AAT AAC GGT TTA TTG TTC
    TTG TCA TGA TTA TTG CCA AAT AAC AAG

                             70
    ATT AAT ACT ACT ATT GCT AGC ATT GCT
    TAA TTA TGA TGA TAA CGA TCG TAA CGA

    75                       80
    GCT AAA GAA GAA GGG GTA C      3'
    CGA TTT CTT CTT CCC            5'
                          (KpnI)
```

**Patentansprüche**
**Patentansprüche für folgende Vertragsstaaten : AT, BE, CH, DE, FR, GB, IT, LI, LU, NL, SE**

**1.** Verbindung der Formel I

B(1-30)-Arg-A(1-21) (I),

in der B(1-30) und A(1-21) die B- bzw. A-Kette des Humaninsulins bedeuten.

**2.** Verbindung der Formel I zur Verwendung als Heilmittel, insbesondere zur Behandlung des Diabetes mellitus.

**3.** Arzneimittel, enthaltend die Verbindung der Formel I.

**4.** Arzneimittel aus einem pharmakologisch unbedenklichen Träger und der Verbindung der Formel I.

**5.** Verwendung der Verbindung der Formel I zur Herstellung der Verbindung der Formel II

```
  S - S
  |   |
A(1 - 21)
  |   |
  S   S                 (II),
  |   |
  S   S
  |   |
B(1 - 30) - Arg
```

in der A(1-21) und B(1-30) die in Anspruch 1 genannte Bedeutung haben und die -S-S-Brücken wie im Insulin angeordnet sind, oder von Insulin, vorzugsweise in einer Eintopfreaktion, worin die Spaltung des Mini-Proinsulins der Formel (I) unter leicht sauren Bedingungen (pH = 6,8) durch geführt wird.

**6.** Verfahren zur Herstellung der Verbindung der Formel I, dadurch gekennzeichnet, daß man eine für diese Verbindung codierende Genstruktur in einer Wirtszelle, vorzugsweise in einem Bakterium oder in einer Hefe, exprimiert und, falls die Genstruktur für ein Fusionsprotein codiert, aus dem erhaltenen Fusionsprotein die Verbindung der Formel I freisetzt.

**7.** DNA, codierend für die Verbindungen der Formel I.

**8.** Genstruktur oder Plasmid, enthaltend die DNA nach Anspruch 7.

**9.** Wirtszelle, vorzugsweise ein Bakterium oder eine Hefe, enthaltend die Genstruktur oder ein Plasmid nach Anspruch 8.

**10.** Fusionsprotein, enthaltend die Verbindung der Formel I, vorzugsweise über ein Brückenglied

- Met - Ile - Glu - Gly - Arg -

an den "Ballastanteil" des Fusionsproteins gebunden.

**Patentansprüche für folgenden Vertragsstaat : ES**

**1.** Verfahren zur Herstellung der Verbindung der Formel I

B(1-30)-Arg-A(1-21) (I),

in der B(1-30) und A(1-21) die B- bzw. A-Kette des Humaninsulins bedeuten, dadurch gekennzeichnet, daß man eine für diese Verbindung codierende Genstruktur in einer Wirtszelle, vorzugsweise in einem Bakterium oder in einer Hefe, exprimiert und, falls die Genstruktur für ein Fusionsprotein codiert, aus dem erhaltenen Fusionsprotein die Verbindung der Formel I freisetzt.

**2.** Verfahren zur Herstellung eines Proteins der Formel I gemäß Anspruch 1, dadurch gekennzeichnet, daß man ein nach Anspruch 1 erhältliches Fusionsprotein enzymatisch spaltet und das Protein der Formel I isoliert.

**3.** Verfahren zur Herstellung der Verbindung der Formel II

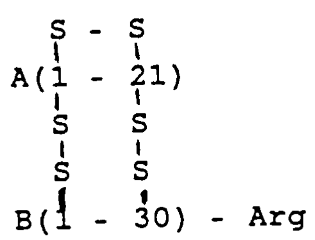

(II),

in der A(1-21) und B(1-30) die vorstehend genannten Bedeutungen haben und die -S-S-Brücken wie im Insulin angeordnet sind, dadurch gekennzeichnet, daß man das nach Anspruch 1 oder 2 erhältliche Protein enzymatisch, unter leicht sauren Bedingungen (pH = 6,8), vorzugsweise mit Trypsin, spaltet.

**4.** Verfahren zur Herstellung von Humaninsulin, dadurch gekennzeichnet, daß man das nach Anspruch 1 oder 2 erhältliches Protein mit Trypsin oder einem analog wirkenden Enzym und mit Carboxypeptidase B oder einem analog wirkenden Enzym in einer Eintopfreaktion unter leicht sauren Bedingungen (pH = 6,8), spaltet.

5. Verfahren zur Herstellung von Humaninsulin, dadurch gekennzeichnet, daß man nach Anspruch 1 ein Fusionsprotein herstellt, dieses spaltet, das Protein der Formel I isoliert, daraus enzymatisch die Verbindung der Formel II herstellt und aus dieser, entweder nach Zwischenisolierung oder vorzugsweise direkt, unter leicht sauren Bedingungen (pH = 6,8), das C-terminale Arginin abspaltet.

6. Verfahren nach Anspruch 1 oder 2, dadurch gekennzeichnet, daß in dem Fusionsprotein das Protein der Formel I an den Ballastanteil über ein Brückenglied der Formel

-Met-Ile-Glu-Gly-Arg-

gebunden ist.

**Patentansprüche für folgenden Vertragsstaat : GR**

1. Verfahren zur Herstellung der Verbindung der Formel I

B(1-30)-Arg-A(1-21) (I),

in der B(1-30) und A(1-21) die B- bzw. A-Kette des Humaninsulins bedeuten, dadurch gekennzeichnet, daß man eine für diese Verbindung codierende Genstruktur in einer Wirtszelle, vorzugsweise in einem Bakterium oder in einer Hefe, exprimiert und, falls die Genstruktur für ein Fusionsprotein codiert, aus dem erhaltenen Fusionsprotein die Verbindung der Formel I freisetzt.

2. Verfahren zur Herstellung eines Proteins der Formel I gemäß Anspruch 1, dadurch gekennzeichnet, daß man ein nach Anspruch 1 erhältliches Fusionsprotein enzymatisch spaltet und das Protein der Formel I isoliert.

3. Verfahren zur Herstellung der Verbindung der Formel II

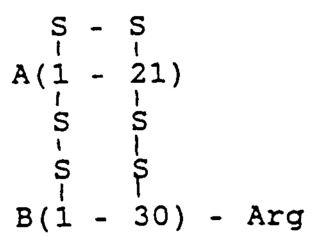

(II),

in der A(1-21) und B(1-30) die vorstehend genannten Bedeutungen haben und die -S-S-Brücken wie im Insulin angeordnet sind, dadurch gekennzeichnet, daß man das nach Anspruch 1 oder 2 erhältliche Protein enzymatisch, unter leicht sauren Bedingungen (pH = 6,8), vorzugsweise mit Trypsin, spaltet.

4. Verfahren zur Herstellung von Humaninsulin, dadurch gekennzeichnet, daß man das nach Anspruch 1 oder 2 erhältliche Protein mit Trypsin oder einem analog wirkenden Enzym und mit Carboxypeptidase B oder einem analog wirkenden Enzym in einer Eintopfreaktion unter leicht sauren Bedingungen (pH = 6,8), spaltet.

5. Verfahren zur Herstellung von Humaninsulin, dadurch gekennzeichnet, daß man nach Anspruch 1 ein Fusionsprotein herstellt, dieses spaltet, das Protein der Formel I isoliert, daraus enzymatisch die Verbindung der Formel II herstellt und aus dieser, entweder nach Zwischenisolierung oder vorzugsweise direkt, unter leicht sauren Bedingungen (pH = 6,8), das C-terminale Arginin abspaltet.

6. Verfahren nach Anspruch 1 oder 2, dadurch gekennzeichnet, daß in dem Fusionsprotein das Protein der Formel I an den Ballastanteil über ein Brückenglied der Formel

-Met-Ile-Glu-Gly-Arg-

gebunden ist.

**7.** DNA, codierend für die Verbindungen der Formel I.

**8.** Genstruktur oder Plasmid, enthaltend die DNA nach Anspruch 7.

**9.** Wirtszelle, vorzugsweise ein Bakterium oder eine Hefe, enthaltend die Genstruktur oder ein Plasmid nach Anspruch 8.

**10.** Fusionsprotein, enthaltend die Verbindung der Formel I, vorzugsweise über ein Brückenglied

- Met - Ile - Glu - Gly - Arg -

an den Ballastanteil des Fusionsproteins gebunden.

**Claims**

**Claims for the following Contracting States : AT, BE, CH, DE, FR, GB, IT, LI, LU, NL, SE**

**1.** The compound of the formula I

B(1-30)-Arg-A(1-21) (I),

in which B(1-30) and A(1-21) denote the B and A chain of human insulin.

**2.** The compound of the formula I for use as a pharmaceutical, in particular for the treatment of diabetes mellitus.

**3.** A pharmaceutical containing the compound of the formula I.

**4.** A pharmaceutical composed of a pharmacologically acceptable excipient and the compound of the formula I.

**5.** The use of the compound of the formula I for the preparation of the compound of the formula II

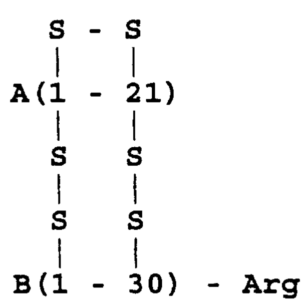

(II),

in which A(1-21) and B(1-30) have the meaning mentioned in claim 1 and the -S-S- bridges are arranged as in insulin, or of insulin, preferably in a one-pot reaction, in which the cleavage of the mini-proinsulin of the formula (I) is carried out under slightly acidic conditions (pH = 6.8).

**6.** A process for the preparation of the compound of the formula I, which comprises expressing a gene structure coding for this compound in a host cell, preferably in a bacterium or in a yeast and, if the gene structure codes for a fusion protein, liberating the compound of the formula I from the fusion protein obtained.

**7.** A DNA, coding for the compound of the formula I.

**8.** A gene structure or plasmid containing the DNA as claimed in claim 7.

**9.** A host cell, preferably a bacterium or a yeast, containing the gene structure or a plasmid as claimed in claim 8.

**10.** A fusion protein containing the compound of the formula I, preferably bonded via a bridging member

- Met - Ile - Glu - Gly - Arg -

to the "ballast component" of the fusion protein.

**Claims for the following Contracting State : ES**

**1.** A process for the preparation of the compound of the formula I

B(1-30)-Arg-A(1-21) (I),

in which B(1-30) and A(1-21) denote the B and A chain of human insulin, which comprises expressing a gene structure coding for this compound in a host cell, preferably in a bacterium or in a yeast and, if the gene structure codes for a fusion protein, liberating the compound of the formula I from the fusion protein obtained.

**2.** The process for the preparation of a protein of the formula I as claimed in claim 1, wherein a fusion protein obtainable as in claim 1 is cleaved enzymatically and the protein of the formula I is isolated.

**3.** A process for the preparation of the compound of the formula II

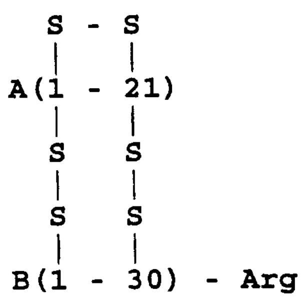

(II),

in which A(1-21) and B(1-30) have the abovementioned meanings and the -S-S- bridges are arranged as in insulin, which comprises cleaving the protein obtainable as in claim 1 or 2 enzymatically under slightly acidic conditions (pH = 6.8), preferably using trypsin.

**4.** A process for the preparation of human insulin, which comprises cleaving the protein obtainable as in claim 1 or 2 with trypsin or an analogously acting enzyme and with carboxypeptidase B or an analogously acting enzyme in a one-pot reaction under slightly acidic conditions (pH = 6.8).

**5.** A process for the preparation of human insulin, which comprises preparing a fusion protein as in claim 1, cleaving it, isolating the protein of the formula I, preparing the compound of the formula II therefrom enzymatically and removing the C-terminal arginine from this, either after intermediate isolation or preferably directly, under slightly acidic conditions (pH = 6.8).

**6.** The process as claimed in claim 1 or 2, wherein, in the fusion protein, the protein of the formula I is bonded via a bridging member of the formula

- Met - Ile - Glu - Gly - Arg -

to the ballast component.

**Claims for the following Contracting State : GR**

**1.** A process for the preparation of the compound of the formula I

B(1-30)-Arg-A(1-21) (I),

in which B(1-30) and A(1-21) denote the B and A chain of human insulin, which comprises expressing a gene structure coding for this compound in a host cell, preferably in a bacterium or in a yeast and, if the gene structure codes for a fusion protein, liberating the compound of the formula I from the fusion protein obtained.

**2.** The process for the preparation of a protein of the formula I as claimed in claim 1, wherein a fusion protein obtainable as in claim 1 is cleaved enzymatically and the protein of the formula I is isolated.

**3.** A process for the preparation of the compound of the formula II

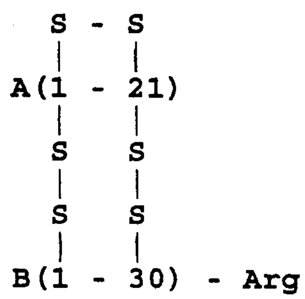

(II),

in which A(1-21) and B(1-30) have the abovementioned meanings and the -S-S- bridges are arranged as in insulin, which comprises cleaving the protein obtainable as in claim 1 or 2 enzymatically under slightly acidic conditions (pH = 6.8), preferably using trypsin.

**4.** A process for the preparation of human insulin, which comprises cleaving the protein obtainable as in claim 1 or 2 with trypsin or an analogously acting enzyme and with carboxypeptidase B or an analogously acting enzyme in a one-pot reaction under slightly acidic conditions (pH = 6.8).

**5.** A process for the preparation of human insulin, which comprises preparing a fusion protein as in claim 1, cleaving it, isolating the protein of the formula I, preparing the compound of the formula II therefrom enzymatically and removing the C-terminal arginine from this, either after intermediate isolation or preferably directly, under slightly acidic conditions (pH = 6.8).

**6.** The process as claimed in claim 1 or 2, wherein, in the fusion protein, the protein of the formula I is bonded via a bridging member of the formula

- Met - Ile - Glu - Gly - Arg -

to the ballast component.

**7.** A DNA coding for the compound of the formula I.

**8.** A gene structure or plasmid containing the DNA as claimed in claim 7.

**9.** A host cell, preferably a bacterium or a yeast, containing the gene structure or a plasmid as claimed in claim 8.

**10.** A fusion protein containing the compound of the formula I, preferably bonded via a bridging member

- Met - Ile - Glu - Gly - Arg -

to the ballast component of the fusion protein.

**Revendications**

**Revendications pour les Etats contractants suivants : AT, BE, CH, DE, FR, GB, IT, LI, LU, NL, SE**

1. Composé de formule I

   B(1-30)-Arg-A-(1-21)  (I)

   dans laquelle B(1-30) et A(1-21) représentent les chaînes B et A de l'insuline humaine.

2. Composé de formule I à utiliser comme agent thérapeutique, en particulier, pour le traitement du diabète sucré.

3. Médicament contenant le composé de formule I.

4. Médicament constitué d'un véhicule pharmacologiquement acceptable et du composé de formule I.

5. Utilisation du composé de formule I pour préparer le composé de formule II

```
  S - S
  |   |
A(1 - 21)
  |   |
  S - S
  |   |                        (II),
  S - S
  |   |
B(1 - 30) - Arg
```

   dans laquelle A(1-21) et B(1-30) ont la signification donnée dans la revendication 1 et les ponts -S-S sont disposés comme dans l'insuline,
   ou l'insuline, de préférence, dans une réaction à un seul réacteur, le clivage de la mini-proinsuline de formule I étant effectuée dans des conditions de légère acidité (pH = 6,8).

6. Procédé pour préparer le composé de formule I, caractérisé en ce que l'on exprime une structure génétique codant pour ce composé dans une cellule-hôte, de préférence, dans une bactérie ou dans une levure, et, dans le cas où la structure génétique code pour une protéine de fusion, on libère le composé de formule I de la protéine de fusion obtenue.

7. ADN codant pour les composés de formule I.

8. Structure génétique ou plasmide contenant l'ADN selon la revendication 7.

9. Cellule-hôte, de préférence, une bactérie ou une levure, contenant la structure génétique ou un plasmide selon la revendication 8.

10. Protéine de fusion contenant le composé de formule I, de préférence, lié par l'intermédiaire d'un élément de pont

    - Met - Ile - Glu - Gly - Arg -

    à la "partie de ballast" de la protéine de fusion.

**Revendications pour l'Etat contractant suivant : ES**

**1.** Procédé pour préparer le composé de formule I

B(1-30)-Arg-A-(1-21) (I)

dans laquelle B(1-30) et A(1-21) représentent les chaînes B et A de l'insuline humaine, caractérisé en ce que l'on exprime une structure génétique codant pour ce composé dans une cellule-hôte, de préférence, dans une bactérie ou dans une levure, et, dans le cas où la structure génétique code pour une protéine de fusion, on libère le composé de formule I de la protéine de fusion obtenue.

**2.** Procédé pour préparer une protéine de formule I selon la revendication 1, caractérisé en ce que l'on clive, par voie enzymatique, une protéine de fusion qu'il est possible d'obtenir selon la revendication 1 et on isole la protéine de formule I.

**3.** Procédé pour préparer le composé de formule II

```
     S - S
     |   |
   A(1 - 21)
     |   |
     S - S
     |   |                        (II),
     S - S
     |   |
   B(1 - 30) - Arg
```

dans laquelle A(1-21) et B(1-30) ont la signification précédemment citée et les ponts -S-S sont disposés comme dans l'insuline, caractérisé en ce que l'on clive , par voie enzymatique, la protéine qu'il est possible d'obtenir selon la revendication 1 ou 2, dans des conditions de légère acidité (pH = 6,8), de préférence, avec de la trypsine.

**4.** Procédé pour préparer de l'insuline humaine, caractérisé en ce que l'on clive une protéine qu'il est possible d'obtenir selon la revendication 1 ou 2, avec de la trypsine, ou une enzyme agissant de manière analogue, et avec de la carboxypeptidase B, ou une enzyme agissant de manière analogue, dans une réaction à un seul réacteur, dans des conditions de légère acidité (pH = 6,8).

**5.** Procédé pour préparer de l'insuline humaine, caractérisé en ce qu'on prépare une protéine de fusion selon la revendication 1, on la clive , on isole la protéine de formule I à partir de laquelle on prépare, par voie enzymatique, le composé de formule II duquel on sépare l'arginine C-terminale, après isolement intermédiaire ou, de préférence, directement, dans des conditions de légère acidité (pH = 6,8).

**6.** Procédé selon la revendication 1 ou 2, caractérisé en ce que, dans la protéine de fusion, la protéine de formule I est liée à la partie de ballast par l'intermédiaire d'un élément de pont de formule

- Met - Ile - Glu - Gly - Arg - .

**Revendications pour l'Etat contractant suivant : GR**

**1.** Procédé pour préparer le composé de formule I

B(1-30)-Arg-A-(1-21) (I)

dans laquelle B(1-30) et A(1-21) représentent les chaînes B et A de l'insuline humaine, caractérisé en

ce que l'on exprime une structure génétique codant pour ce composé dans une cellule-hôte, de préférence, dans une bactérie ou dans une levure, et, dans le cas où la structure génétique code pour une protéine de fusion, on libère le composé de formule I de la protéine de fusion obtenue.

**2.** Procédé pour préparer une protéine de formule I selon la revendication 1, caractérisé en ce que l'on clive par voie enzymatique, une protéine de fusion qu'il est possible d'obtenir selon la revendication 1 et on isole la protéine de formule I.

**3.** Procédé pour préparer le composé de formule II

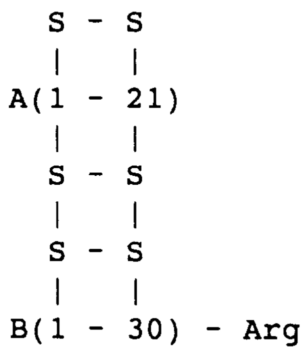

(II),

dans laquelle A(1-21) et B(1-30) ont la signification précédemment citée et les ponts -S-S sont disposés comme dans l'insuline, caractérisé en ce que clive par voie enzymatique, la protéine qu'il est possible d'obtenir selon la revendication 1 ou 2, dans des conditions de légère acidité (pH = 6,8), de préférence, avec de la trypsine.

**4.** Procédé pour préparer de l'insuline humaine, caractérisé en ce que l'on clive une protéine qu'il est possible d'obtenir selon la revendication 1 ou 2, avec de la trypsine, ou une enzyme agissant de manière analogue, et avec de la carboxypeptidase B, ou une enzyme agissant de manière analogue, dans une réaction à un seul réacteur, dans des conditions de légère acidité (pH = 6,8).

**5.** Procédé pour préparer de l'insuline humaine, caractérisé en ce qu'on prépare une protéine de fusion selon la revendication 1, on la clive, on isole la protéine de formule I à partir de laquelle on prépare, par voie enzymatique, le composé de formule II duquel on sépare l'arginine C-terminale, après isolement intermédiaire ou, de préférence, directement, dans des conditions de légère acidité (pH = 6,8).

**6.** Procédé selon la revendication 1 ou 2, caractérisé en ce que, dans la protéine de fusion, la protéine de formule I est liée à la "partie de ballast" par l'intermédiaire d'un élément de pont de formule

- Met - Ile - Glu - Gly - Arg - .

**7.** ADN codant pour les composés de formule I.

**8.** Structure génétique ou plasmide contenant l'ADN selon la revendication 7.

**9.** Cellule-hôte, de préférence, une bactérie ou une levure, contenant la structure génétique ou un plasmide selon la revendication 8.

**10.** Protéine de fusion contenant le composé de formule I, de préférence, lié par l'intermédiaire d'un élément de pont

- Met - Ile - Glu - Gly - Arg -

à la "partie de ballast" de la protéine de fusion.

FIG.1

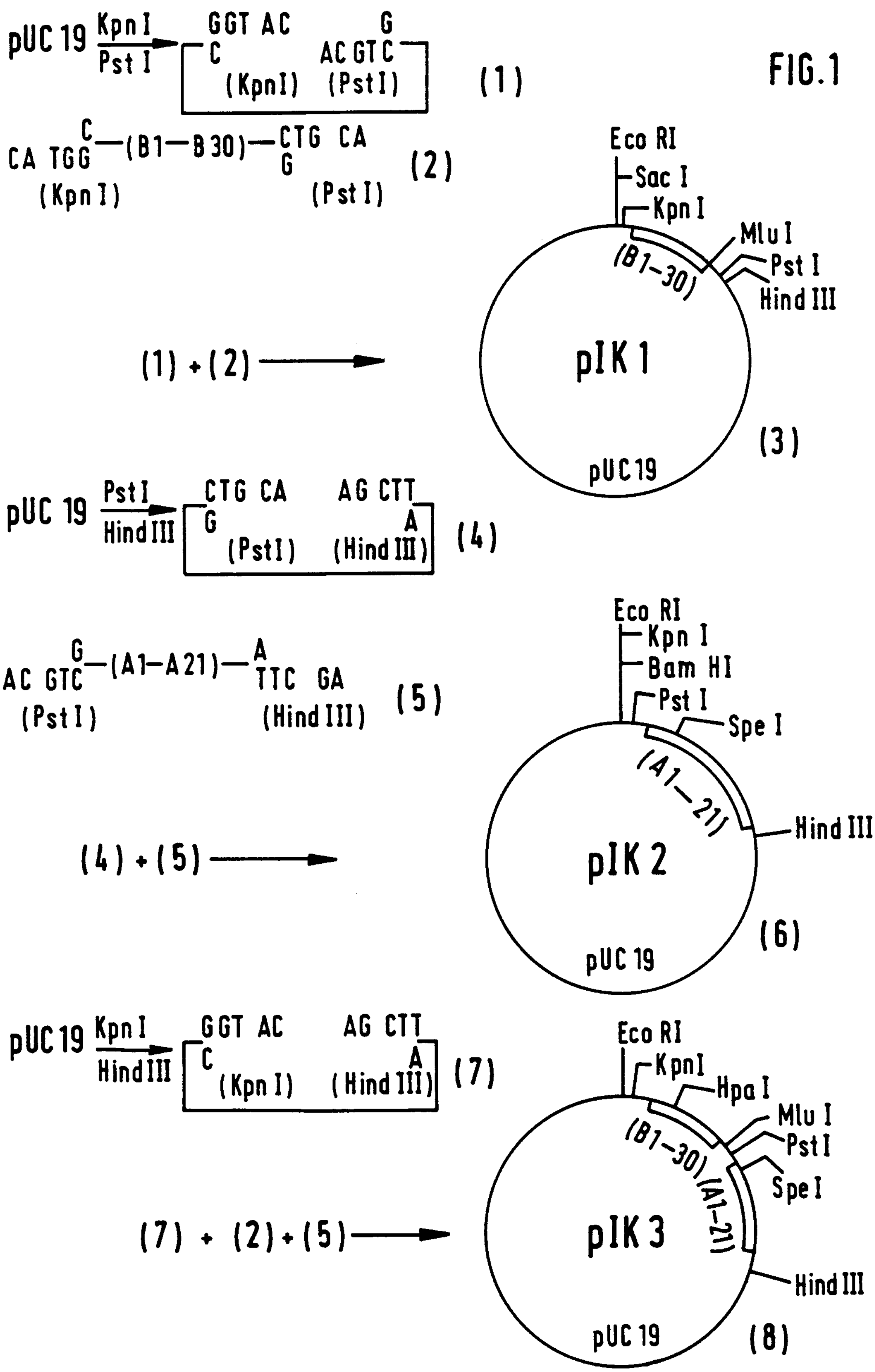
pUC 19
Kpn I
Pst I
GGT AC
C
G
AC GTC
(KpnI) (PstI)
(1)
C
CA TGG —(B1—B30)— CTG CA
G
(Kpn I) (Pst I)
(2)
(1) + (2)
Eco RI
Sac I
Kpn I
Mlu I
Pst I
Hind III
(B1–30)
pIK 1
pUC19
(3)
pUC 19
Pst I
Hind III
CTG CA
G
AG CTT
A
(PstI) (Hind III)
(4)
G
AC GTC —(A1—A21)— TTC GA
A
(PstI) (Hind III)
(5)
(4) + (5)
Eco RI
Kpn I
Bam HI
Pst I
Spe I
(A1—21)
Hind III
pIK 2
pUC 19
(6)
pUC19
Kpn I
Hind III
GGT AC
C
AG CTT
A
(Kpn I) (Hind III)
(7)
(7) + (2) + (5)
Eco RI
KpnI
Hpa I
Mlu I
Pst I
Spe I
(B1–30)
(A1–21)
Hind III
pIK 3
pUC 19
(8)

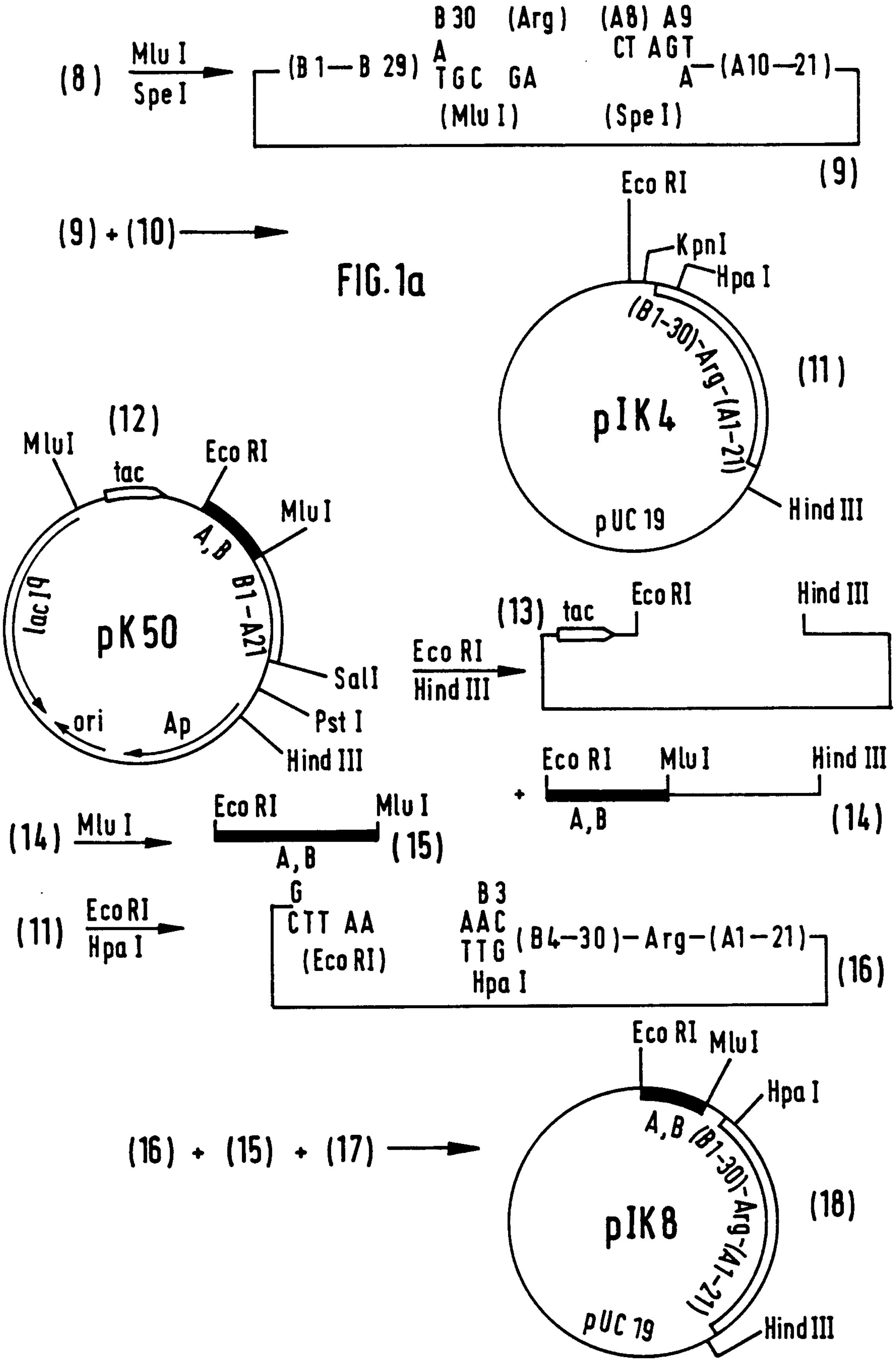
(8)
Mlu I
Spe I
B 30
(Arg)
(A8) A9
A
CT AGT
(B 1—B 29)
TGC GA
A
(A10—21)
(Mlu I)
(Spe I)
(9)
(9) + (10)
Eco RI
KpnI
Hpa I
(B1-30)-Arg-(A1-21)
(11)
pIK4
pUC 19
Hind III
(12)
MluI
tac
Eco RI
MluI
A,B
B1-A21
lacIq
pK50
SalI
Pst I
Hind III
ori
Ap
(13)
tac
Eco RI
Hind III
Eco RI
Hind III
Eco RI
MluI
Hind III
A,B
(14)
(14)
Mlu I
Eco RI
Mlu I
A,B
(15)
(11)
EcoRI
Hpa I
G
CTT AA
(Eco RI)
B3
AAC
TTG
Hpa I
(B4-30)-Arg-(A1-21)
(16)
(16) + (15) + (17)
Eco RI
MluI
Hpa I
A,B
(B1-30)-Arg-(A1-21)
(18)
pIK8
pUC 19
HindIII

FIG. 1a

FIG. 1b

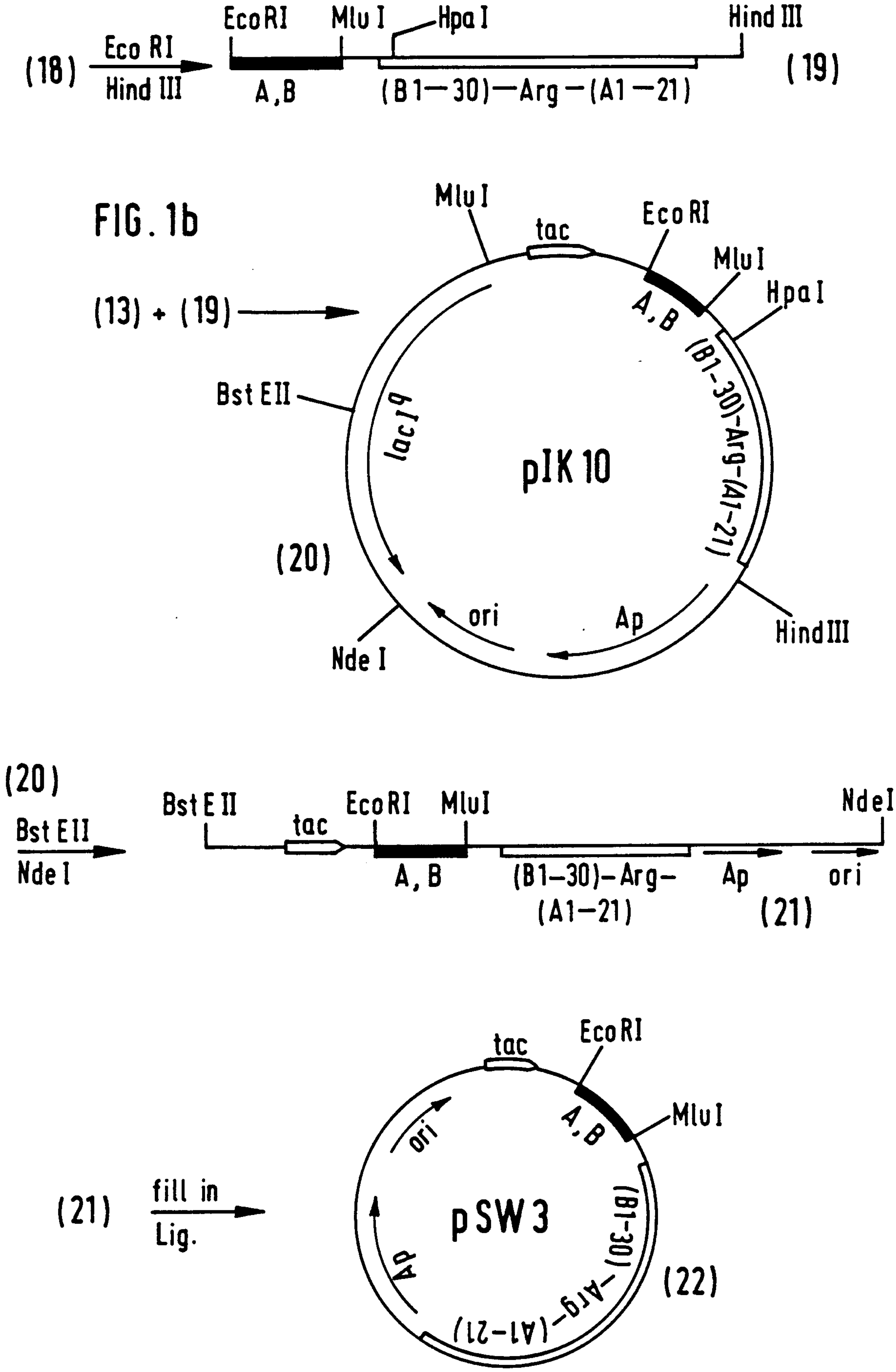
(18)
Eco RI
Hind III
EcoRI
Mlu I
Hpa I
Hind III
A,B
(B1—30)—Arg—(A1—21)
(19)
(13) + (19)
MluI
tac
EcoRI
MluI
HpaI
A,B
(B1-30)-Arg-(A1-21)
BstEII
lacIq
pIK10
(20)
ori
Ap
NdeI
HindIII
(20)
BstEII
NdeI
BstEII
tac
EcoRI
MluI
NdeI
A, B
(B1—30)—Arg—
(A1—21)
Ap
ori
(21)
(21)
fill in
Lig.
tac
EcoRI
MluI
A,B
ori
pSW3
Ap
(B1-30)-Arg-(A1-21)
(22)

FIG. 2

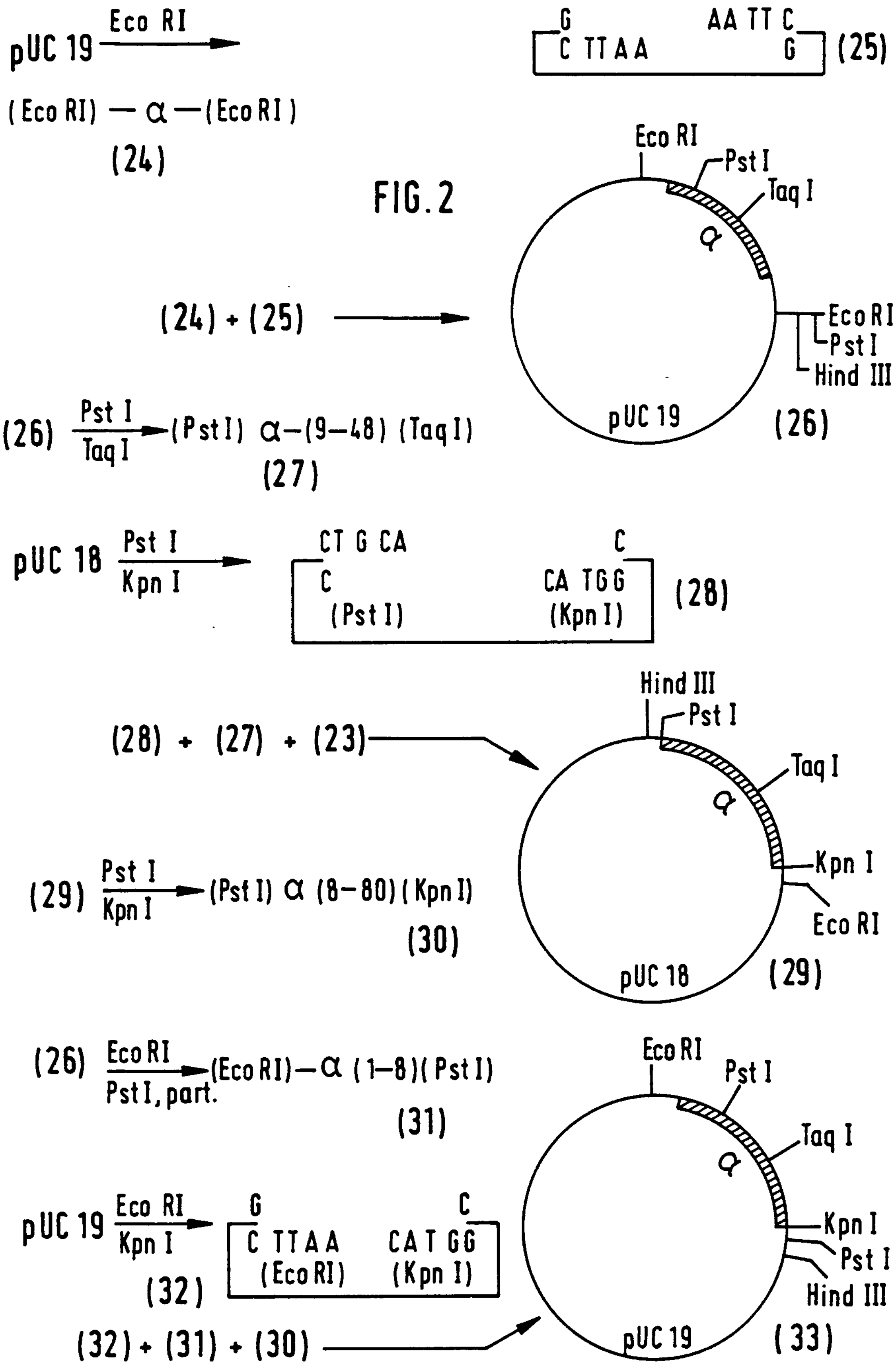
pUC 19 Eco RI
G AA TT C
C TT A A G (25)
(Eco RI) — α — (Eco RI)
(24)
Eco RI
Pst I
Taq I
α
(24) + (25)
Eco RI
Pst I
Hind III
pUC 19
(26)
(26) Pst I Taq I (Pst I) α–(9–48) (Taq I)
(27)
pUC 18 Pst I Kpn I
CT G CA C
C CA TG G
(Pst I) (Kpn I)
(28)
Hind III
Pst I
(28) + (27) + (23)
Taq I
α
Kpn I
Eco RI
(29) Pst I Kpn I (Pst I) α (8–80) (Kpn I)
(30)
pUC 18
(29)
(26) Eco RI Pst I, part. (Eco RI)–α (1–8) (Pst I)
(31)
Eco RI
Pst I
Taq I
α
pUC 19 Eco RI Kpn I
G C
C TT A A CA T GG
(Eco RI) (Kpn I)
(32)
Kpn I
Pst I
Hind III
(32) + (31) + (30)
pUC 19
(33)

FIG. 2a

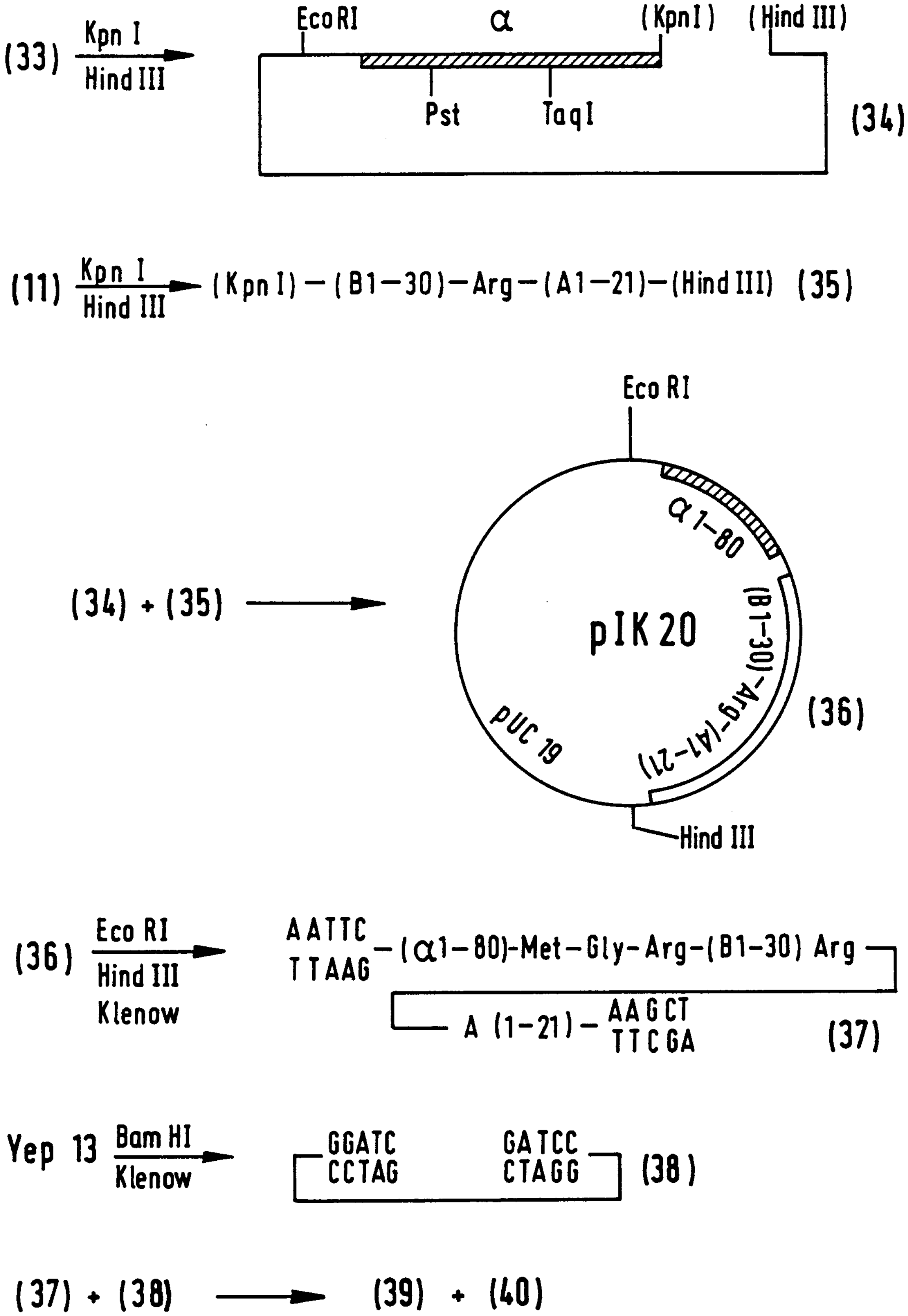
(33) Kpn I / Hind III
EcoRI
α
(KpnI)
(Hind III)
Pst
TaqI
(34)
(11) Kpn I / Hind III
(Kpn I) – (B1–30) – Arg – (A1–21) – (Hind III) (35)
(34) + (35)
Eco RI
α 1–80
pIK 20
(B1–30)–Arg–(A1–21)
(36)
pUC 19
Hind III
(36) Eco RI / Hind III / Klenow
AATTC
TTAAG
– (α1–80)-Met–Gly–Arg–(B1–30) Arg
A (1–21) –
AAGCT
TTCGA
(37)
Yep 13 Bam HI / Klenow
GGATC
CCTAG
GATCC
CTAGG
(38)
(37) + (38)
(39) + (40)

FIG. 2b

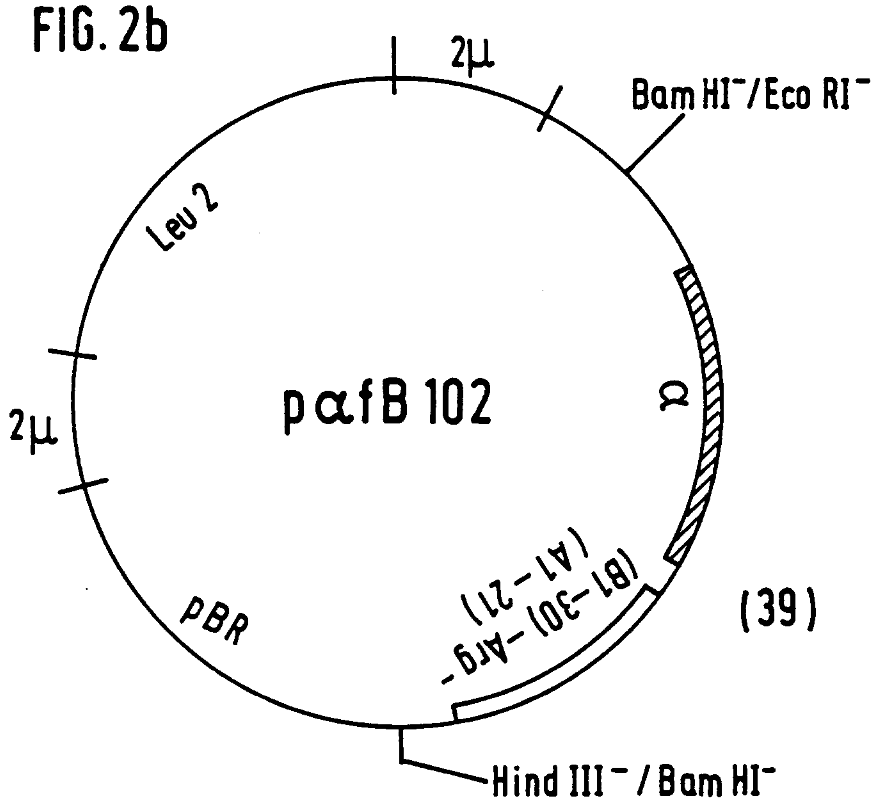
2μ
Bam HI⁻/Eco RI⁻
Leu 2
pαfB 102
α
2μ
(B1-30)-Arg-
(A1-21)
pBR
Hind III⁻/Bam HI⁻

(39)

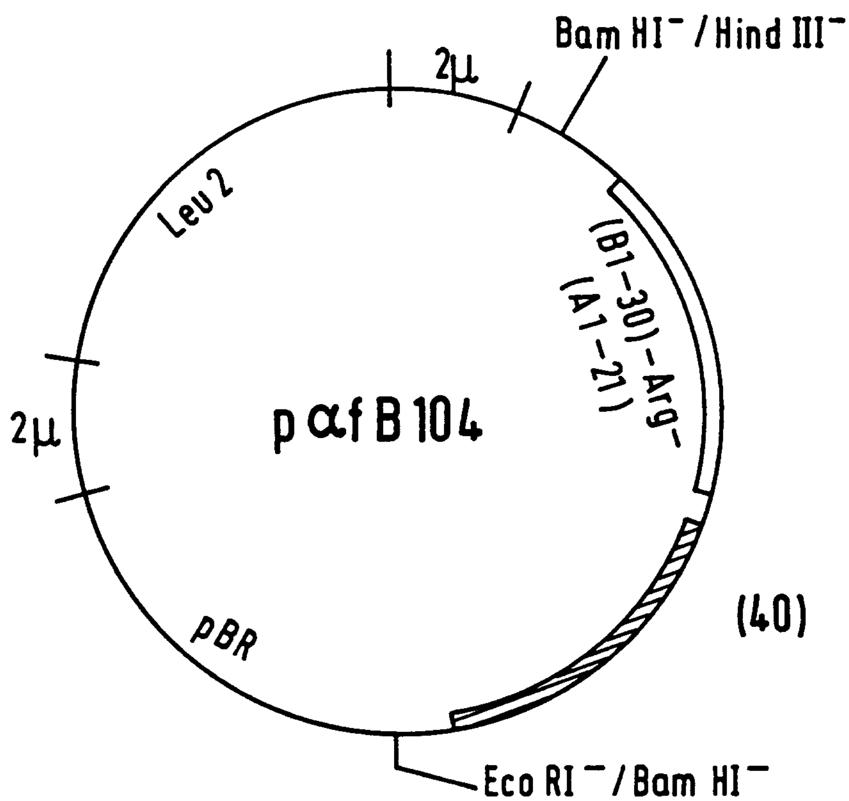
Bam HI⁻/Hind III⁻
2μ
Leu 2
(B1-30)-Arg-
(A1-21)
pαfB 104
2μ
pBR
Eco RI⁻/Bam HI⁻

(40)